(19) Europäisches Patentamt
European Patent Office
Office européen des brevets

(11) **EP 2 209 529 B1**

(12) # EUROPEAN PATENT SPECIFICATION

(45) Date of publication and mention
of the grant of the patent:
**19.09.2018 Bulletin 2018/38**

(21) Application number: **08836801.4**

(22) Date of filing: **10.10.2008**

(51) Int Cl.:
*A61P 35/00* (2006.01)   *A61K 31/5377* (2006.01)

(86) International application number:
**PCT/EP2008/063605**

(87) International publication number:
**WO 2009/047323 (16.04.2009 Gazette 2009/16)**

(54) **Isoxazole compound for the treatment of breast cancer**

Isoxazolverbindung zur Behandlung von Brustkrebs

Composé isoxazole pour le traitement du cancer du sein

(84) Designated Contracting States:
**AT BE BG CH CY CZ DE DK EE ES FI FR GB GR
HR HU IE IS IT LI LT LU LV MC MT NL NO PL PT
RO SE SI SK TR**

(30) Priority: **12.10.2007 EP 07118421**

(43) Date of publication of application:
**28.07.2010 Bulletin 2010/30**

(60) Divisional application:
**10174276.5 / 2 263 751**
**18187344.9**

(73) Proprietor: **Vernalis (R&D) Limited
Winnersh
Wokingham
Berkshire RG41 5RD (GB)**

(72) Inventors:
• **CHENE, Patrick**
**F-68100 Mulhouse (FR)**
• **GARCIA-ECHEVERRIA, Carlos**
**CH-4052 Basel (CH)**
• **JENSEN, Michael Rugaard**
**CH-4054 Basel (CH)**
• **QUADT, Cornelia**
**CH-4123 Allschwil (CH)**
• **RADIMERSKI, Thomas**
**CH-4492 Tecknau (CH)**
• **SCHOEPFER, Joseph**
**CH-4125 Riehen (CH)**

(74) Representative: **Gill Jennings & Every LLP
The Broadgate Tower
20 Primrose Street
London EC2A 2ES (GB)**

(56) References cited:
**WO-A-2004/072051     WO-A-2006/113498
US-A1- 2007 105 862**

• **DATABASE ADISCTI [Online] ADIS; 13
September 2007 (2007-09-13), US NATIONAL
INSTITUTES OF HEALTH: "AUY922 : therapeutic
use Advanced breast cancer Phase I/II trial in
patients with either HER2-positive or oestrogen
receptor-positive locally advanced or metastatic
disease" XP002466385 retrieved from STN
Database accession no. 2007:8882**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

The invention relates to the use of 5-(2,4-Dihydroxy-5-isopropyl-phenyl)-4-(4-morpholin-4-ylmethyl-phenyl)-isoxazole-3-carboxylic acid ethylamide or a pharmaceutically acceptable salt, hydrate or solvate thereof, for the manufacture of pharmaceutical compositions for use in the treatment of cancer of the breast, and to 5-(2,4-Dihydroxy-5-isopropyl-phenyl)-4-(4-morpholin-4-ylmethyl-phenyl)-isoxazole-3-carboxylic acid ethylamide or a pharmaceutically acceptable salt, hydrate or solvate thereof, for use in the treatment of cancer of the breast.

Management of cancer of the breast is a major problem.

Heat shock protein 90 (Hsp90) is recognized as a new anti-cancer target. Hsp90 is a ubiquitous, highly abundant (1-2% of the total cellular protein), essential protein which functions as a molecular chaperone to ensure the conformational stability, shape and function of client proteins. Inhibition of its intrinsic ATPase activity of Hsp90 disrupts the Hsp90-client protein interaction resulting in their degradation via the ubiquitin proteasome pathway. A subset of Hsp90 client proteins, such as Raf, AKT, CDK4 and the EGFR family including ErbB2 are oncogenic signaling molecules critically involved in cell growth, differentiation and apoptosis, processes which are fundamentaly important in cancer cells. The simultaneous degradation of multiple oncoproteins is believed to produce the anti-tumor effects observed with Hsp90 inhibitors.

The Hsp90 family of chaperones is comprised of four members: Hsp90$\alpha$ and Hsp90$\beta$ both located in the cytosol, GRP94 in the endoplasmic reticulum, and TRAP1 in the mitochondria (Csermely et al., 1998). Hsp90 is the most abundant cellular chaperone, constituting about 1% - 2% of total protein (Jakob and Buchner, 1994). Among the stress proteins, Hsp90 is unique because it is not required for the biogenesis of most polypeptides (Nathan et al., 1997). Its cellular targets, also called client proteins, are conformationally labile signal transducers that play a critical role in growth control, cell survival and tissue development (Pratt and Toft, 2003).

Hsp90 chaperones, which possess a conserved ATP-binding site at their N-terminal domain (Chene, 2002) belong to a small ATPase sub-family known as the DNA Gyrase, Hsp90, Histidine Kinase and MutL (GHKL) sub-family (Dutta and Inouye, 2000). The chaperoning (folding) activity of Hsp90 depends on its ATPase activity which is weak for the isolated enzyme. However, it has been shown that the ATPase activity of Hsp90 is enhanced upon its association with proteins known as co-chaperones (Kamal et al., 2003). Therefore, *in vivo,* Hsp90 proteins work as subunits of large, dynamic protein complexes. Hsp90 is essential for eukaryotic cell survival and is overexpressed in many tumors.

The use of various Hsp90 inhibitors for the treatment of cancer, has been previously described in US 2007/105862 A1 and WO 2006/113498 A. 5-(2,4-Dihydroxy-5-isopropylphenyl)-4-(4-morpholin-4-ylmethyl-phenyl)-isoxazole-3-carboxylic acid ethylamide is an Hsp90 inhibitor. Its synthesis is described in example 78 of WO 2004/072051 A.

Surprisingly it has now been found that 5-(2,4-Dihydroxy-5-isopropyl-phenyl)-4-(4-morpholin-4-ylmethyl-phenyl)-isoxazole-3-carboxylic acid ethylamide or a pharmaceutically acceptable salt, hydrate or solvate thereof, is useful in the treatment of cancer of the breast.

Accordingly the present invention provides the use of 5-(2,4-Dihydroxy-5-isopropyl-phenyl)-4-(4-morpholin-4-ylmethyl-phenyl)-isoxazole-3-carboxylic acid ethylamide or a pharmaceutically acceptable salt, hydrate or solvate thereof, for the manufacture of pharmaceutical compositions for use in the treatment of cancer of the breast.

In a further aspect the present invention provides 5-(2,4-Dihydroxy-5-isopropyl-phenyl)-4-(4-morpholin-4-ylmethyl-phenyl)-isoxazole-3-carboxylic acid ethylamide or a pharmaceutically acceptable salt, hydrate or solvate thereof, for use in treating cancer of the breast.

In a further aspect the present invention provides a pharmaceutical preparation for the treatment of cancer of the breast comprising 5-(2,4-Dihydroxy-5-isopropyl-phenyl)-4-(4-morpholin-4-ylmethyl-phenyl)-isoxazole-3-carboxylic acid ethylamide or a pharmaceutically acceptable salt, hydrate or solvate thereof, and at least one pharmaceutically acceptable carrier.

Depending on species, age, individual condition, mode of administration, and the clinical picture in question, effective doses for example weekly doses of about 2 to 300 mg, preferably 50 to 160 mg of 5-(2,4-Dihydroxy-5-isopropyl-phenyl)-4-(4-morpholin-4-ylmethyl-phenyl)-isoxazole-3-carboxylic acid ethylamide or a tautomer thereof or a pharmaceutically acceptable salt or a hydrate or a solvate are administered to a human.

Following is a description by way of example only.

**Example 1: *In vitro* effects of 5-(2,4-Dihydroxy-5-isopropyl-phenyl)-4-(4-morpholin-4-ylmethyl-phenyl)-isoxazole-3-carboxylic acid ethylamide (AUY922) on a panel of tumor derived cell lines.**

Thirty-seven cancer derived cell lines are used (BT474, MDA-MB-361, MDA-MB-453, SKBr3, T47D, MCF7, MDA-MB-231, MDA-MB-468, SK-MEL-5, A375, MALME-3M, SK-MEL-28, WM266.4, RPMI8226, U266, BE, Colo205, HCT116, HT29, MAWI, RKO, U87MG, HN5, RPMI-8226, A549, MV522, NCI-H1299, NCI-H460, 41M, A2780, CH1, NCI-N87, SKOV3, PC3, MO7e, GIST882 and Baf3) to test the effect of 5-(2,4-Dihydroxy-5-isopropyl-phenyl)-4-(4-morpholin-4-ylmethyl-phenyl)-isoxazole-3-carboxylic acid ethylamide. Cell lines are commercially available from American Type Culture Collection (ATCC). These cell lines cover the following 12 cancer or tumor types: breast, melanoma, multiple myeloma (MM), colon, glioblastoma, head & neck, leukemia, lung, ovarian, prostate, stomach and gastrointestinal stromal tumour (GIST). After division and medium change, cells from stock culture are seeded on cell plates and cultured for about 18 hours to allow cell growth and attachment before starting the assay. On the first day of the assay, 5-(2,4-Dihydroxy-5-isopropyl-phenyl)-4-(4-morpholin-4-ylmethyl-phenyl)-isoxazole-3-carboxylic acid ethylamide is added to the medium at various concentrations up to 10 $\mu$. Cells are cultured up to 72 or 96 hours and cell proliferation is determined using commercially available cell proliferation kits.

Table 1 shows the concentrations (nM) of 5-(2,4-Dihydroxy-5-isopropyl-phenyl)-4-(4-morpholin-4-ylmethyl-phenyl)-isoxazole-3-carboxylic acid ethylamide which inhibit cell proliferation by 50% ($IC_{50}$). The cells were continually exposed to 5-(2,4-Dihydroxy-5-isopropyl-phenyl)-4-(4-morpholin-4-ylmethyl-phenyl)-isoxazole-3-carboxylic acid ethylamide for either 72 or 96 hours and cell growth was determined by commercially available kits based on either SRB, Alamar blue, methylene blue or WST-1 methods.

**Table 1**

| Tumor type | Cell line | $IC_{50}$ (nM) |
| --- | --- | --- |
| Breast | BT474 | 2.8 |
| | MDA-MB-361 | 6 |
| | MDA-MB-453 | 3.9 |
| | SKBr3 | 2.3 |
| | T47D | 2.6 |
| | MCF7 | 2.3 |
| | MDA-MB-231 | 7.7 |
| | MDA-MB-468 | 3.5 |
| Melanoma | SK-MEL-5 | 3 |
| | A375 | 3 |
| | MALME-3M | 7.7 |
| | SK-MEL-28 | 8 |
| | WM266.4 | 6.2 |
| Multiple myeloma (MM) | RPMI8226 | 36.7 |
| | U266 | 23.3 |
| Colon | BE | 2.8 |
| | Colo205 | 6.2 |
| | HCT116 | 16 |
| | HT29 | 30 |
| | MAWI | 50 |
| | RKO | 3.1 |
| Glioblastoma | U87MG | 6 |
| Head & Neck | HN5 | 8 |
| Leukemia | RPMI-8226 | 6.3 |
| Lung | A549 | 11.7 |
| | MV522 | 8.1 |
| | NCI-H1299 | 5.7 |
| | NCI-H460 | 14 |
| Ovarian | 41M | 3 |
| | A2780 | 6.1 |
| | CH1 | 2.8 |
| | SKOV3 | 3.7 |
| Prostate | PC3 | 5 |
| Stomach | NCI-N87 | 0.2 |

(continued)

| Tumor type | Cell line | IC$_{50}$ (nM) |
|---|---|---|
| Gastrointestinal stromal | MO7e | 10.6 |
| tumour (GIST) | GIST882 | 6.2 |
| | Baf3 | 22.4 |

Data relating to tumor types other than breast is outside the scope of the claims.

**Example 2: *In vitro* effects of 5-(2,4-Dihydroxy-5-isopropyl-phenyl)-4-(4-morpholin-4-ylmethyl-phenyl)-isoxazole-3-carboxylic acid ethylamide (AUY922) on a panel of primary human tumor cells.**

The anticancer activity of 5-(2,4-Dihydroxy-5-isopropyl-phenyl)-4-(4-morpholin-4-ylmethyl-phenyl)-isoxazole-3-carboxylic acid ethylamide is evaluated in 30 human tumor xenografts *in vitro* using a clonogenic assay. In this assay, human cells derived from cancer patients are evaluated for the capacity of 5-(2,4-Dihydroxy-5-isopropyl-phenyl)-4-(4-morpholin-4-ylmethyl-phenyl)-isoxazole-3-carboxylic acid ethylamide to inhibit the formation of 3 dimensional colonies. These consist of tumor cells that possess the potential for anchorage independent growth in semisolid medium. The tumor xenografts which have never been cultured in cell culture plastic dishes are isolated from nude mice. Tumor cell suspensions are prepared and incubated in 24 well plates containing layers of soft agar. Under these conditions a special subpopulation of cells selectively grows to colonies. 5-(2,4-Dihydroxy-5-isopropyl-phenyl)-4-(4-morpholin-4-ylmethyl-phenyl)-isoxazole-3-carboxylic acid ethylamide was tested in 6 concentrations up to 10 $\mu$M. The tumor test panel comprises 1 to 6 models of 10 different human tumor or cancer types, which were bladder cancer, colon, liver, non small cell lung (adeno, squamous epithelium and large cell), small cell lung, mammary, ovary, pancreatic, melanoma and pleuramesothelioma. Antitumor effects are recorded as inhibition of colony formation in relation to untreated controls. The concentration which results in 50% reduction in colony formation (IC$_{50}$) are shown in Table 2. Further information on the method has been published (Burger et al., 2004; Fiebig et al., 2004; Smith et al., 2005).

Table 2 shows the concentration (nM) of 5-(2,4-Dihydroxy-5-isopropyl-phenyl)-4-(4-morpholin-4-ylmethyl-phenyl)-isoxazole-3-carboxylic acid ethylamide which inhibits colony formation by 50% (IC$_{50}$). The cells are continually exposed to 5-(2,4-Dihydroxy-5-isopropylphenyl)-4-(4-morpholin-4-ylmethyl-phenyl)-isoxazole-3-carboxylic acid ethylamide and colony formation is determined.

**Table 2**

| Tumor Type | Tumor model | Histology | IC50 (nM) |
|---|---|---|---|
| Bladder | BXF 1218 | Transitional cell carcinoma | 27 |
| | BXF 1228 | Transitional cell carcinoma | 630 |
| Colon | CXF 1103 | Adeno carcinoma | 13 |
| | CXF 158 | Adeno carcinoma | 369 |
| | CXF 1729 | Carcinoma | 467 |
| | CXF 1784 | Carcinoma | 418 |
| | CXF 609 | Adeno carcinoma | 55 |
| Liver | LIXF 575 | Hepatocellular carcinoma | 34 |
| Lung, non- small cell | LXFA 297 | Adeno carcinoma | 28 |
| | LXFA 526 | Adeno carcinoma | 5 |
| | LXFA 629 | Adeno carcinoma | 35 |
| | LXFA 983 | Adeno carcinoma | 126 |
| | LXFE 1422 | Squamous cell carcinoma | 48 |
| | LXFL 1647 | Large cell lung carcinoma | 34 |
| Lung, small cell | LXFS 615 | Small cell lung carcinoma | 30 |
| | LXFS 650 | Small cell lung carcinoma | 2 |
| Breast | MAXF 1162 | Invasive ductal carcinoma | 304 |
| | MAXF 1322 | Pap. adeno carcinoma | 29 |
| | MAXF 1384 | Adeno carcinoma | 209 |

(continued)

| Tumor Type | Tumor model | Histology | IC50 (nM) |
|---|---|---|---|
| | MAXF 401 | Pap. adeno carcinoma | 78 |
| | MAXF 583 | Ductual adeno carcinoma | 333 |
| Melanoma | MEXF 1539 | Melanoma | 3 |
| | MEXF 462 | Amelanotic melanoma | 24 |
| | MEXF 535 | Amelanotic melanoma | 43 |
| | MEXF 672 | Amelanotic melanoma | 18 |
| | MEXF 989 | Amelanotic melanoma | 2 |
| Ovary | OVXF 1353 | Adeno carcinoma | 26 |
| | OVXF 1544 | Carcinoma | 53 |
| Pancreas | PAXF 1657 | Adeno carcinoma | 39 |
| Pleuramesothelioma | PXF 1118 | Biphasic pleuramesothelioma | 223 |

Data relating to tumor types other than breast is outside the scope of the claims.

**Example 3: Antitumor effect of 5-(2,4-Dihydroxy-5-isopropyl-phenyl)-4-(4-morpholin-4-ylmethyl-phenyl)-isoxazole-3-carboxylic acid ethylamide (AUY922) in the human breast cancer model BT-474**

The estrogen receptor positive cell line BT-474 was initially isolated from a human breast ductal carcinoma established from a solid, invasive ductal carcinoma of the breast obtained from a 60-year-old woman (ATCC number HTB-20). The cells are grown in DMEM high glucose (4.5 g/l) supplemented with 10% FCS, 200 mM L-glutamine and 1% sodium pyruvate.

In preparation for cell inoculation, each mouse is subcutaneously implanted on the upper dorsal side with a 17$\beta$-Estradiol pellet (25 $\mu$g/day; 90 day release) using a trocar needle. BT-474 cells (5x10^6) are injected in 200 $\mu$l Matrigel:HBSS (1:1 vol) (BD Matrigel™ Basement Membrane Matrix). The injection site is subcutaneously in the right flank. Treatment with AUY922 is initiated when the average tumor volume reached approximately 100 mm$^3$. Tumor growth is monitored at regular intervals. The xenograft tumor sizes are measured manually with calipers and the tumor volume is estimated using the formula: (W x L x H x $\pi$/6), where width (W), height (H) and length (L) are the three largest diameters.

Results are presented as mean $\pm$ SEM. Tumor data are analyzed by ANOVA with post hoc Dunnet's test for comparison of treatment versus control group. As a measure of efficacy the %T/C value is calculated at the end of the experiment according to:

$$(\Delta \text{tumor volume}_{treated}/\Delta \text{tumor volume}_{control})*100$$

where $\Delta$tumor volumes represent the mean tumor volume on the evaluation day minus the mean tumor volume at the start of the experiment.

The antitumor effect of AUY922 is evaluated in the BT-474 xenograft model. In this study, the treatment period is 21 days. Each group consists of eight tumor bearing animals. At the end of the study, the tumor sizes in the treatment groups are compared to those of the vehicle treated groups and the effect is expressed as %T/C. Statistically significant reduction of tumor sizes are observed when AUY922 is administered once per week at 17-25 mg/kg (Table 3).

**Table 3: Effect of AUY922 on BT-474 xenograft growth**

| Compound | Dose, schedule, route | T/C (%) | $\Delta$Tumor volume (mm$^3$) |
|---|---|---|---|
| Vehicle control | 10 ml/kg, qw, i.v. | 100 | 528 $\pm$ 123 |
| AUY922 | 8.3 mg/kg, qw, i.v. | 43 | 229 $\pm$ 73 |
| AUY922 | 17 mg/kg, qw, i.v. | 9 | 46 $\pm$ 27* |
| AUY922 | 25 mg/kg, qw, i.v. | 3 | 15 $\pm$ 23* |
| * P < 0.05; one-way ANOVA *post hoc* Dunnet's test. | | | |

**Example 4: Antitumor effect of 5-(2,4-Dihydroxy-5-isopropyl-phenyl)-4-(4-morpholin-4-ylmethyl-phenyl)-isoxazole-3-carboxylic acid ethylamide (AUY922) in the rat breast cancer model BN-472**

The transplantable rat breast cancer tumor BN472 is serially passaged as fragments in female syngeneic Brown Norway rats. The injection site is orthotopically in the mammary fat pad. Treatment with AUY922 is initiated when the average tumor volume reaches approximately 100 mm$^3$. Tumor growth is monitored at regular intervals. The xenograft tumor sizes are measured manually with calipers and the tumor volume is estimated using the formula: (W x L$^2$ x $\pi$/6), where width (W) and height (H) are the two largest diameters. Results are presented as mean $\pm$ SEM. Tumor data were analyzed by ANOVA with *post hoc* Dunnet's test for comparison of treatment versus control group. As a measure of efficacy the %T/C value is calculated at the end of the experiment according to:

$$(\Delta\text{tumor volume}_{treated}/\Delta\text{tumor volume}_{control})*100$$

where $\Delta$tumor volumes represent the mean tumor volume on the evaluation day minus the mean tumor volume at the start of the experiment.

The antitumor effect of AUY922 is evaluated in the BN472 xenograft model. Each group consists of seven tumor bearing animals. At the end of the study, the tumor sizes in the treatment groups are compared to those of the vehicle treated groups and the effect is expressed as %T/C. Statistically significant reduction of tumor sizes is observed when AUY922 was administered once per week at 50 mg/kg (Table 4).

**Table 4: Effect of AUY922 on BN472 xenograft growth**

| Compound | Dose, schedule, route | T/C (%) | $\Delta$Tumor volume (mm$^3$) |
|---|---|---|---|
| Vehicle control | 2 ml/kg, qw, i.v. | 100 | 5569 $\pm$ 1639 |
| AUY922 | 25 mg/kg, qw, i.v. | 78 | 4357 $\pm$ 1338 |
| AUY922 | 50 mg/kg, qw, i.v. | 21 | 1148 $\pm$ 152* |
| * P < 0.05; one-way ANOVA *post hoc* Dunnet's test. | | | |

**Example 5: Antitumor effect of 5-(2,4-Dihydroxy-5-isopropyl-phenyl)-4-(4-morpholin-4-ylmethyl-phenyl)-isoxazole-3-carboxylic acid ethylamide (AUY922) in the rat pancreatic cancer model CA20948**

The transplantable rat pancreatic tumor CA20948 is serially passaged as cell homogenates in male syngeneic Lewis rats. The injection site is subcutaneously on the right flank. Treatment with AUY922 is initiated when the average tumor volume reaches approximately 100 mm$^3$. Tumor growth is monitored at regular intervals. The xenograft tumor sizes is measured manually with calipers and the tumor volume is estimated using the formula: (W x L$^2$ x $\pi$/6), where width (W) and height (H) are the two largest diameters.

Results are presented as mean $\pm$ SEM. Tumor data were analyzed by ANOVA with *post hoc* Dunnet's test for comparison of treatment versus control group. As a measure of efficacy the %T/C value is calculated at the end of the experiment according to:

$$(\Delta\text{tumor volume}_{treated}/\Delta\text{tumor volume}_{control})*100$$

where $\Delta$tumor volumes represent the mean tumor volume on the evaluation day minus the mean tumor volume at the start of the experiment.

The antitumor effect of AUY922 is evaluated in the CA20948 xenograft model. Each group consisted of six tumor bearing animals. At the end of the study, the tumor sizes in the treatment groups are compared to those of the vehicle treated groups and the effect is expressed as %T/C. Statistically significant reduction of tumor sizes is observed when AUY922 is administered once per week at 50 and 75 mg/kg (Table 5).

**Table 5: Effect of AUY922 on CA20948 xenograft growth**

| Compound | Dose, schedule, route | T/C (%) | $\Delta$Tumor volume (mm$^3$) |
|---|---|---|---|
| Vehicle control | 2 ml/kg, qw, i.v. | 100 | 23267 $\pm$ 7810 |

(continued)

| Compound | Dose, schedule, route | T/C (%) | $\Delta$Tumor volume (mm$^3$) |
|---|---|---|---|
| AUY922 | 50 mg/kg, qw, i.v. | 30 | 7090 $\pm$ 2553* |
| AUY922 | 75 mg/kg, qw, i.v | 21 | 4796 $\pm$ 1354* |
| * P < 0.05; one-way ANOVA *post hoc* Dunnet's test. | | | |

Example 5 is outside the scope of the claims.

## Claims

1. The use of 5-(2,4-Dihydroxy-5-isopropyl-phenyl)-4-(4-morpholin-4-ylmethyl-phenyl)isoxazole-3-carboxylic acid ethylamide or a pharmaceutically acceptable salt, hydrate or solvate thereof, for the manufacture of a pharmaceutical composition for the treatment of cancer of the breast.

2. The use of 5-(2,4-Dihydroxy-5-isopropyl-phenyl)-4-(4-morpholin-4-ylmethyl-phenyl)isoxazole-3-carboxylic acid ethylamide, or a pharmaceutically acceptable salt thereof, according to claim 1.

3. 5-(2,4-Dihydroxy-5-isopropyl-phenyl)-4-(4-morpholin-4-ylmethyl-phenyl)-isoxazole-3-carboxylic acid ethylamide or a pharmaceutically acceptable salt, hydrate or solvate thereof, for use in treating cancer of the breast.

4. 5-(2,4-Dihydroxy-5-isopropyl-phenyl)-4-(4-morpholin-4-ylmethyl-phenyl)-isoxazole-3-carboxylic acid ethylamide, or a pharmaceutically acceptable salt thereof, for use according to claim 3.

5. A pharmaceutical preparation comprising 5-(2,4-Dihydroxy-5-isopropyl-phenyl)-4-(4-morpholin-4-ylmethyl-phenyl)-isoxazole-3-carboxylic acid ethylamide or a pharmaceutically acceptable salt, hydrate or solvate thereof and at least one pharmaceutically acceptable carrier, for use in the treatment of cancer of the breast.

## Patentansprüche

1. Verwendung von 5-(2,4-Dihydroxy-5-isopropylphenyl)-4-(4-morpholin-4-ylmethylphenyl)isoxazol-3-carbonsäureethylamid oder eines pharmazeutisch verträglichen Salzes, Hydrats oder Solvats davon für die Herstellung einer pharmazeutischen Zusammensetzung für die Behandlung von Krebs der Brust.

2. Verwendung von 5-(2,4-Dihydroxy-5-isopropylphenyl)-4-(4-morpholin-4-ylmethylphenyl)isoxazol-3-carbonsäureethylamid oder eines pharmazeutisch verträglichen Salzes davon nach Anspruch 1.

3. 5-(2,4-Dihydroxy-5-isopropylphenyl)-4-(4-morpholin-4-ylmethylphenyl)isoxazol-3-carbonsäureethylamid oder ein pharmazeutisch verträgliches Salz, Hydrat oder Solvat davon für die Verwendung bei der Behandlung von Krebs der Brust.

4. 5-(2,4-Dihydroxy-5-isopropylphenyl)-4-(4-morpholin-4-ylmethylphenyl)isoxazol-3-carbonsäureethylamid oder ein pharmazeutisch verträgliches Salz davon für die Verwendung nach Anspruch 3.

5. Pharmazeutisches Präparat, das 5-(2,4-Dihydroxy-5-isopropylphenyl)-4-(4-morpholin-4-ylmethylphenyl)isoxazol-3-carbonsäureethylamid oder ein pharmazeutisch verträgliches Salz, Hydrat oder Solvat davon und mindestens einen pharmazeutisch verträglichen Träger umfasst, für die Verwendung bei der Behandlung von Krebs der Brust.

## Revendications

1. Utilisation de l'éthylamide de l'acide 5-(2,4-dihydroxy-5-isopropyl-phényl)-4-(4-morpholin-4-ylméthyl-phényl)isoxazol-3-carboxylique ou d'un sel, hydrate ou solvate pharmaceutiquement acceptable de celui-ci dans la fabrication d'une composition pharmaceutique pour le traitement du cancer du sein.

**2.** Utilisation de l'éthylamide de l'acide 5-(2,4-dihydroxy-5-isopropyl-phényl)-4-(4-morpholin-4-ylméthyl-phényl)isoxazol-3-carboxylique ou d'un sel pharmaceutiquement acceptable de celui-ci selon la revendication 1.

**3.** Éthylamide de l'acide 5-(2,4-dihydroxy-5-isopropyl-phényl)-4-(4-morpholin-4-ylméthyl-phényl)isoxazol-3-carboxylique ou sel, hydrate ou solvate pharmaceutiquement acceptable de celui-ci pour une utilisation dans le traitement du cancer du sein.

**4.** Éthylamide de l'acide 5-(2,4-dihydroxy-5-isopropyl-phényl)-4-(4-morpholin-4-ylméthyl-phényl)isoxazol-3-carboxylique ou sel pharmaceutiquement acceptable de celui-ci pour une utilisation selon la revendication 3.

**5.** Préparation pharmaceutique comprenant l'éthylamide de l'acide 5-(2,4-dihydroxy-5-isopropyl-phényl)-4-(4-morpholin-4-ylméthyl-phényl)isoxazol-3-carboxylique ou un sel, hydrate ou solvate pharmaceutiquement acceptable de celui-ci et au moins un véhicule pharmaceutiquement acceptable pour une utilisation dans le traitement du cancer du sein.

**EP 2 209 529 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2007105862 A1
- WO 2006113498 A
- WO 2004072051 A